# EUROPEAN PATENT SPECIFICATION

(11) **EP 1 210 445 B1**
(45) Date of publication and mention of the grant of the patent: **07.03.2007**
(21) Application number: 00958787.4
(22) Date of filing: 06.09.2000
(51) Int. Cl.: C12N 15/67, C12N 1/21, A61K 39/02, C12N 1/20

(54) **RECOMBINANT MICROORGANISMS**
REKOMBINANTE MIKROORGANISMEN
MICRO-ORGANISMES DE RECOMBINAISON

(30) Priority: 10.09.1999 GB 9921275; 12.07.2000 GB 0017000
(43) Date of publication of application: 05.06.2002
(73) Proprietor: THE SECRETARY OF STATE FOR DEFENCE, Salisbury SP4 0JQ (GB)
(72) Inventor: TITBALL, Richard William, Salisbury, Wiltshire SP4 0JQ (GB); BULLIFENT, Helen Lisa, Salisbury, Wiltshire SP4 0JQ (GB)
(74) Representative: Beckham, Robert William
(86) International application number: PCT/GB2000/003402
(87) International publication number: WO 2001/019974

(56) References cited:
- WO-A-96/28551
- TITBALL RICHARD W ET AL: "Expression of the Yersinia pestis capsular antigen (F1 antigen) on the surface of an aroA mutant of Salmonella typhimurium induces high levels of protection against plague." INFECTION AND IMMUNITY, vol. 65, no. 5, 1997, pages 1926-1930, XP002164415 ISSN: 0019-9567
- HOHMANN ELIZABETH L ET AL: "Macrophage-inducible expression of a model antigen in Salmonella typhimurium enhances immunogenicity." PROCEEDINGS OF THE NATIONAL ACADEMY OF SCIENCES OF THE UNITED STATES, vol. 92, no. 7, 1995, pages 2904-2908, XP002164416 1995 ISSN: 0027-8424
- ROBERTS MARK ET AL: "Oral vaccination against tetanus: Comparison of the immunogenetics of Salmonella strains expressing fragment C from the nirB and htrA promoters." INFECTION AND IMMUNITY, vol. 66, no. 7, July 1998 (1998-07), pages 3080-3087, XP002164417 ISSN: 0019-9567 cited in the application
- MCSORLEY STEPHEN J ET AL: "Vaccine efficacy of Salmonella strains expressing glycoprotein 63 with different promoters." INFECTION AND IMMUNITY, vol. 65, no. 1, 1997, pages 171-178, XP002164418 ISSN: 0019-9567 cited in the application
- BULLIFENT HELEN L ET AL: "Antibody responses to Yersinia pestis F1-antigen expressed in Salmonella typhimurium aroA from in vivo-inducible promoters." VACCINE, vol. 18, no. 24, 1 June 2000 (2000-06-01), pages 2668-2676, XP002164419 ISSN: 0264-410X

## Description

The present invention relates to recombinant microorganisms, in particular gut-colonising organisms, which are useful for example in the delivery of antigenic material and thus form the basis of vaccines. Vaccines comprising these organisms and promoter sequences for use in them form a further aspect of the invention.

Attenuated mutants of *Salmonella typhi* (e.g. *aroA, aroC, htrA*) are currently being evaluated as live, oral vaccines against typhoid fever (Tacket CO, et al., Infect. Immun. 1997;65:452-6). These mutants have also attracted attention as carriers for guest (vaccine) antigens but suitable animal models for testing these vaccines are not available. In view of this, many workers have used *Salmonella typhimurium aroA* expressing guest antigens for investigating the immune responses induced after oral vaccination of mice.

The unregulated expression of foreign genes within *Salmonella* species such as *S. typhimurium* can lead to plasmid instability, yet the stable expression of the guest antigen at the appropriate site in the body is necessary for the induction of a protective response. One approach to promote the stable expression of guest antigens involves the chromosomal integration of the heterologous gene. However, this may reduce the immune response because of gene dosage effects (Covone M G, et al., Infect. Immun. 1998;66:224-31).

The balanced lethal system (Curtiss R III, et al., Res. Microbiol. 1990;141:797-805, Nakayama K, et al., Bio/Technology 1988;6:693-97) relies on the complementation of a lethal mutation by a plasmid which also encodes the guest antigen. Whilst this ensures retention of the plasmid, the gene encoding the guest antigen itself may be deleted. An alternative approach involves the use of promoters which are induced within host tissues to direct guest antigen expression at that site. Because the gene is only expressed after certain environmental cues have been recognised, this approach might reduce the selective pressure towards deleting the gene.

This solution to the problem of expression of guest antigens has also been identified by other workers. A variety of antigens have been expressed in *S. typhimurium* from the *nirB* promoter which is upregulated under anaerobic conditions and within host cells (Oxer M D, et al. Nucleic Acids Res. 1991;19:2889-92). Guest antigens delivered using the *nirB* promoter system induce superior responses than the same antigens delivered from a constitutive promoter. In addition, the *nirB* promoter-driven genes were maintained more effectively in the *Salmonella* host strain. More recently, it has been shown that the *htrA* and *osmC* promoter can be used to direct expression of guest antigens in *Salmonella* (McSorley S J, et al., Infect. Immun. 1997;65: 171-78, Roberts M, et al., Infect. Immun. 1998; 66:3080-87). However, it is likely that these promoters will not be suited to the expression of all guest antigens.

Immunisation with the F1-antigen of *Y. pestis* has previously been shown to induce an antibody-mediated protective response against plague (Green M, et al., FEMS Microbiology and Immunology, 1998;23:107-13) and so to be useful in a vaccine (WO 96/28551). We have previously shown that the F1-antigen can be expressed in *S. typhimurium* (Oyston P C F, et al., Infect. Immun. 1995;63:563-68, Titball R W; et al., Infect. Immum. 1997;65:1926-30). The antigenic properties of F1-antigen have been exploited to investigate the ways in which different promoters, which are induced at different sites in the body, can be used to induce different antibody responses to guest antigens expressed in *S. typhimurium.* It is known that the invasion and spread of *S. typhimurium* within the host is accompanied by the expression of different subsets of genes which are involved in processes such as attachment and invasion, penetration of the epithelium and the infection of deep lymphoid tissue.

The OmpR/EnvZ two component regulatory system responds to changes in the osmotic strength and pH within *S*. *typhimurium* (Foster J W, et al., Microbiology 1994;140:341-52). It has been suggested that this system might play a role in allowing the bacterium to survive in the gut by regulating the expression of outer membrane porins such as OmpC (Pratt L A, et al., American Society for Microbiology, ASM Press, Washington DC, 1995, pp105-27, Nikaido H, et al., Cellular and Molecular Biology. American Society for Microbiology, Washington DC. 1987, pp7-22, Garcia véscovi E. et al., Cell. 1996;84:165-74).

The PhoP/PhoQ two-component regulatory system controls virulence properties such as survival within macrophages, resistance to host defence antimicrobial peptides and acid pH, invasion of epithelial cells, the formation of spacious vacuoles and the processing and presentation of antigens by activated macrophages (Miller S I. et al., Proc. Natl. Acad, Sci USA 1989;86:5054-58, Fields P I, et al., Science 1989;243:1059-62, Pegues D A, et al., Mol. Microbiol. 1995;17:169-81, Wick M J, et al., Mol. Microbiol. 1995;16:465-76), in response to environmental magnesium concentration (Garcia Véscovi E. et al., Cell. 1996;84:165-74). Over forty genes are regulated by this system in *S*. *typhimurium* (Soncini F C, et al., J. Bacteriol. 1996;178:5092-99) including the *phoP* gene, which is autoregulated (Soncini F C, et al., J. Bacteriol. 1995;177:4364-71) and the *pagC* gene which encodes an envelope protein required for survival in the macrophage (Alpuche-Aranda C M, et al., Proc. Natl. Acad. Sci. USA 1992;89:10079-83).

Attenuation of Salmonella by partial deletion of the pagC gene and fusion to a heterologous protein is described in USP 5,733,760. Hohmann et al. Proc. Natl. Acad. Sci. (1995) 92: 2904-2908 describes macrophage inducible expression of a model antigen in S. typhimurium as a means of enhancing immunogenicity.

The applicants have however found that certain promoters can be used advantageously in such systems to drive high levels of expression of heterologous proteins, in particular in mucosal cells.

Thus, the present invention provides a method of enhancing expression of a desired protein at mucosal effector sites, said method comprising placing the protein to be expressed under the control of a promoter having SEQ ID NO 2 or SEQ ID NO 3 or a fragment or variant or any of these which has promoter activity, and causing expression in mucosal cells.

Further according to the present invention, there is provided a construct comprising a promoter selected from the P_{phoP} and P_{pagC} or fragments or variants thereof which can act as promoters, operatively interconnected with a nucleic acid which encodes a protein, able to induce a protective immune response against an organism, in a mammal to which it is administered, wherein said construct contains no further elements of the phoP or pagC gene.

The present invention further provides a recombinant gut-colonising microorganism which comprises a promoter selected from the P_{phoP} and P_{pagC} or fragments or variants thereof which can act as promoters, said promoter being operatively interconnected with a nucleic acid which encodes a heterologous protein, able to induce a protective immune response against a different organism, in a mammal to which it is administered.

In particular, the microorganism has been transformed with the construct described above.

The term "heterologous protein" refers to proteins which are not native to the microorganism strain.

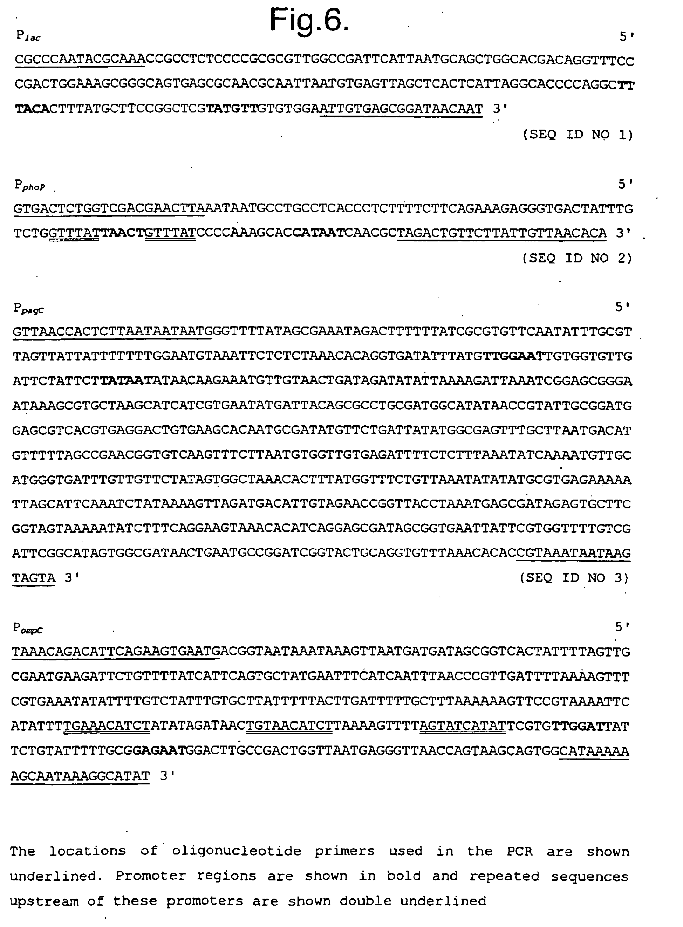

The promoters (P_{phoP} and P_{pagC}) which are included in the organisms of the invention are induced at different stages in the infection process, and hence at different sites in the body. This approach allows the induction of different immune responses which provide protection against pathogens which colonise different host cell compartments. The sequence of these promoters has been elucidated previously, and these are given hereinafter in Figure 6 as SEQ ID NOS 2 and SEQ ID NO 3 respectively. The sequence of the P_{ompC} promoter is given as SEQ ID NO 4.

Their expression has been compared to that of the constitutively expressed *lacZ* gene promoter. As a result, recombinant gut-colonising microorganisms wherein antigen expression is driven by P_{phoP} promoter forms a preferred embodiment of the invention.

The development of effective vaccines against pathogens is dependent not only on the identification of the appropriate protective antigens but also on the induction of an immune response at the site in the body which provides maximum protection against disease. For some pathogens, serum antibody provides protection against disease. However, many pathogens enter the body at a mucosal surface and protection against these diseases might therefore be dependent on the induction of mucosal immune responses. The *Salmonella* vaccine vector system is ideally suited to the delivery of many vaccine antigens since the vaccine delivery mechanism accurately mimics the natural disease, entering the body via the gut.

Thus in particular embodiment, the recombinant gut-colonising microorganism comprises a *Salmonella spp.* such as *Salmonella typhimurium* or *Salmonella typhi.*

The recombinant *Salmonella* evaluated here showed significant differences in their abilities to induce mucosal IgA antibody responses. Serum IgA levels were not a good predictor of mucosal IgA levels, in accordance with the general findings by other workers that these responses are not well correlated [Lu FX et al., Infect. Immun. 1999;67:6321-8; Russell MW et al., Infect. Immun. 1991;59:4061-70; and Wenneras C et al., Infect. Immun. 1999; 67:6231-41]. After oral dosing all of the recombinant *Salmonella* would have entered the body via M-cells, and, if sufficient antigen was subsequently presented to immune effector cells then mucosal antibody responses would be expected. The finding that mucosal antibody in the gut was induced only after immunisation with recombinant *Salmonella* expressing F1-antigen from the *phoP* or *pagC* gene promoters suggests that these promoters directed high-level expression of F1-antigen within GALT. Peyer's patch cells taken from mice immunised with SL3261 / pP*_{pagC}*-F1 or SL3261 / pP*_{phoP}*-F1 produced the highest levels of IgA supporting this suggestion.

Immunisation with *Salmonella* containing pP*_{phoP}*-F1 also resulted in detectable IgA antibody in the lungs. This is in accordance with the finding that this recombinant also induced the highest levels of IgA in the gut and might indicate that the SL3261 / pP*_{phoP}*-F1 was more effective than SL3261 / pP*_{pagC}*-F1 in inducing long-term expression of IgA.

Recombinant gut-colonising microorganisms of the invention are suitably attenuated so that the host does not experience significant harmful effects as a result of infection by the microorganism. Examples of attenuated mutants include *aro* mutants such as *aroA* and *aroC* mutants, apartate β-semi-aldehyde dehydrogenase (ASD) mutants, purine biosynthesis mutants, branched chain amino acid biosynthesis mutants, galactose epimerase (*galE*) mutants, regulatory mutants such as *phoP* and *phoQ* mutants, htrA serine protease mutants and adenyl cyclase mutants. Particular attenuated strains of *Salmonella,* such as *Salmonella typhi* include *aroA, aroC and htrA* mutants or triple mutants including all three mutations.

Recombinant gut-colonising microorganism as described above can be used to deliver a variety of antigenic agents which can be used to induce a protective immune response against a wide range of pathogens. Pathogens which may be targeted in this way are those of humans or animals and include those listed in the Health and Safety Executive: "Categorisation of Biological Agents according to Hazard and Category of Containment", HMSO, ISBN 0717610381. Particular examples of antigenic agents which may be included in the recombinant organisms of the invention include those protective against tetanus such as tetanus toxin H_{C} fragment, those protective against Botulinum such as *botulinum* toxin H_{C} fragment, those protective against *Bacillus anthracis* such as *Bacillus anthracis* protective antigen (PA), those protective against *Bordetella pertussis* such as *Bordetella pertussis* P69 antigen, those protective against *Schistoma mansoni* such as *Schistoma mansoni* glutathibne-S-transferase, those protective against cholera such as Fibrio cholera β subunit, those protective against Herpes simplex virus (HSV) such as HSV glycoprotein D, those protective against HIV infection such as HIV envelope protein, and those protective against *Escherischia coli* such as *E. coli* LTB subunit or *E. coli* K88 antigen. Other suitable antigenic agents as those protective against *Mycrobacterium tuberculosis* as well as agents which protects or enhances anti-tumour immunity. In particular, it has been found that where the heterologous protein, is able to induce a protective immune response against *Yersinia pestis,* useful protective immunity is found. Examples of antigens which can produce such as response include the F1-antigen of *Yersinia pestis* or an antigenic fragment or variant thereof, or the V-antigen of *Yersinia pestis* or combinations thereof as described in WO 96/28551.

The expression "variant" refers to sequences of amino acids which differ from the base sequence from which they are derived in that one or more amino acids within the sequence are substituted for other amino acids. Amino acid substitutions may be regarded as "conservative" where an amino acid is replaced with a different amino acid with broadly similar properties. Non-conservative substitutions are where amino acids are replaced with amino acids of a different type. Broadly speaking, fewer non-conservative substitutions will be possible without altering the biological activity of the polypeptide. Suitably variants will be at least 60% homologous, preferably at least 75% homologous, and more preferably at least 90% homologous to the base sequence. Homology in this instance can be determined using in particular the Needleman-Wunsch algorithm with gap penalty of 8 using a standard PAM scoring matrix (Needleman S.B. and Wunsch C.D., J. Mol Biol. 1970, vol 48, 443-453).

The recombinant gut-colonising microorganisms described above are thus particularly suitable for use in the preparation of vaccines for therapeutic or prophylactic purposes, where they may be combined with a pharmaceutically acceptable carrier or diluent, as would be understood in the art.

In particular, the vaccines will be formulated so that they are adapted for oral administration and that the microorganism remains viable throughout any storage period. Thus they may preferably be in a form liquid form such as aqueous or oily suspensions, emulsions, syrups or elixirs.

The size of the dose for therapeutic or prophylactic purposes of will vary according to a wide variety of factors including the nature of the protective immune response sought, the nature of the antigen being employed, the severity of the conditions, the dosage regime in terms of primary and secondary boosting, the age and sex of the animal or patient and the gut-colonising ability of the particular microorganism used. In general however, a dosage of microorganism in the range of from 10° to 10⁹ cfu will be administered as a single dosage.

vaccine compositions may further comprise a buffer such as a bicarbonate buffer, in order to neutralise stomach acid.

Thus invention may be used in a method of inducing a protective immune response against a pathogen in a mammal, said method comprising administering to said mammal a recombinant gut-colonising microorganism which comprises a promoter selected from the P_{phoP} and P_{pagC} or fragments or variants thereof which can act as promoters, said promoter being operatively interconnected with a nucleic acid which encodes an antigen protein, able to induce a protective immune response against said pathogen, in a mammal to which it is administered.

In yet a further aspect, the invention provides the use of a promoter selected from P_{phoP} and P_{pagC} in the production of a vaccine comprising a recombinant gut-colonising organism.

The promoters used in this study are induced at specific sites in the body. They are preferably cloned into the microorganism in a low copy number vector, because high copy number plasmids have been shown to be unstable in *S typhimurium* (Coulson N M, et al., Microb Pathog. 1994;16:305-11).
The PhoP gene would be expected to be expressed at a basal level from the *PhoPp2* promoter and upregulated in the phagosome of host cells as a result of activation of the PhoPp1 promoter (Soncini F C, et al., J. Bacteriol. 1995;177:4364-71). The PhoP / PhoQ regulatory system has been shown to regulate the expression of a variety of genes including pagC, and to be important for survival in macrophages (Miller S I. et al., Proc. Natl. Acad, Sci USA 1989;86:5054-58, Wick M J, et al., Mol. Microbiol. 1995;16:465-76).

Genes regulated by the PhoP/PhoQ system are also important for the virulence of orally delivered bacteria (Galan J E, et al., Microb Pathog. 1989;6:433-43). Expression of the *ompC* is gene is upregulated under conditions of high osmotic strength (Foster J W, et al., Microbiology 1994;140:341-52, Nikaido H, et al., Cellular and Molecular Biology. American Society for Microbiology, Washington DC. 1987, pp7-22), such as those found within the gut, under control of the OmpR/EnvZ regulatory system (Pratt L A, et al., American Society for Microbiology, ASM Press, Washington DC, 1995, pp105-27).

Whilst the different plasmids in *S*. *typhimurium* SL3261 were stable in vitro, there were marked differences in the stability of the plasmids in bacteria which had been delivered to mice by the oral route. Bacteria expressing the F1-antigen from the *PagC* promoter showed a much reduced ability to colonise mesenteric lymph nodes and appeared incapable of further invasion of the host. It is possible that the additional copies of the *pagC* promoter and upstream regulatory regions titrated out the available PhoP activator within the cell, and that this prevented the bacterium from responding to the environmental changes encountered after uptake by M-cells. However, recombinant *Salmonella* containing the P*_{phoP}*-F1 plasmid did not show a similar inability to invade the host.

This finding might be in accordance with the suggestion that *pho*P expression is only partially autoregulated by the *pho*P gene product (Fields P I, et al., Science 1989;243:1059-62). Additionally, it is possible that the high level of expression of F1-antigen from the *pag*C promoter in vivo placed a lethal metabolic load on the host bacterium.

These promoters are regulated by a variety of environmental stimuli in a manner which is not fully defined. Therefore, it is difficult to make meaningful comparisons of the strengths of these promoters in vitro. Thus, *in vivo* testing of these promoters to identify those most suitable for use for the expression of guest antigens has been carried out.

All of the recombinant *Salmonella* induced similar levels of antibody against the whole bacterium. This finding was unexpected for bacteria containing pP*_{pagC}*-F1, since these bacteria were unable to invade deep host tissues and were recovered only at low levels from mesenteric lymph nodes. This recombinant *Salmonella* also induced IgG and IgA antibody against the F1-antigen. This suggests that the initial interaction of the bacteria with M-cells is critical in determining the immune response to the bacterium and to guest antigens. This conclusion is supported by the finding that *Salmonella* containing pP*_{pag}*C-F1 induced mucosal antibody to the F1-antigen whereas bacteria expressing the F1-antigen expressed from the *lac*Z or *omp*C promoters failed to induce mucosal responses. Therefore, the measurement of the colonisation of spleen or liver tissues, as an indicator of vaccine potential of recombinant *Salmonella,* may not always be useful.

Similar conclusions were reached by Covone et al. (Covone M G, et al., Infect. Immun. 1998;66:224-31) who showed that effective delivery of the LTK63 guest antigen to the immune system was effective only when the antigen was delivered during the early stages of invasion and by McSorley et al. (McSorley S J, et al., Infect. Immun. 1997;65:171-78) who showed that recombinant *Salmonella* expressing glycoprotein 63 from the *osm*C promoter were unable to invade tissue beyond the mediastinal lymph nodes, yet induced protection against *Leishmania major.* This might also explain why killed *Salmonella* with or without guest antigens, which are clearly not able to invade deep host tissues, are able to induce an immune response (Thatte J, et al., Int. Immunol. 1993;5:1431-36).

The ability of *Salmonella* expressing P*_{phoP}*-F1 to induce mucosal antibody responses to the F1-antigen in both the gut and the lungs, whereas a constitutive promoter (P*_{lacZ}*) failed to induce such responses clearly demonstrates the utility of in vivo induced promoters for the induction of appropriate antibody responses. This promoter system will be particularly useful for other applications where a mucosal antibody response is important for protection against disease.

The invention will now be particularly described by way of Example with reference to the accompanying diagrammatic drawings in which :
Figure 1 is a plasmid diagram illustrating plasmids used in the preparation of microorganisms in accordance with the invention;
Figure 2 is a graph showing the levels of colonisation of spleen tissues of mice, 11 days after dosing with recombinant microorganisms of the invention;
Figure 3 shows graphs illustrating IgG serum antibody levels in mice to the carrier bacterium, (Fig 3a) and to the F1 antigen (Fig 3b), 21, 28 and 98 days after immunisation;
Figure 4 is a graph showing the isotype of the F1 antibody found in mice serum on day 98, where the blank column represents the amount of the IgG1ₐ and the shaded column represents the IgG2ₐ isotype in all groups of immunised animals:
Figure 5 is a graph showing the levels of circulating IgA antibody to F1-antigen or the levels of IgA antibody to F1-antigen in gut (blank column) or lung (shaded column) wash samples;
Figure 6 shows sequences of promoters used in the evaluation of the invention; and
Figure 7 shows the results of elispot analysis of Peyer's patch cells and in particular the IgA response against F1 antigen (Figure 7a) and Salmonella (Figure 7b).

### Example 1

### Preparation of Bacterial strains, cultivation and enzymes

*Escherichia coli* strain JM109 and *S. typhimurium* strains LB5010 (rm* *galE),* SL3261 (*aroA*) or SL1344 (a mouse-virulent strain; (Zhang X, et al., Infect. Immun. 1997;65:5381-7) were cultured on L-agar or in L-broth, supplemented with ampicillin (05µg/ml) where appropriate. Enzymes used for DNA cloning and amplification procedures were obtained from BCL limited (Lewes, Sussex, UK). PCR reactions were carried out using a Perkin Elmer 9600 (P.E. Applied Biosystems, Warrington, UK) thermal cycler with cycle conditions of 95°C, 5 min, followed by 50 cycles of 95°C, 5s; 45°C, 5s; 72°C, 5s, followed by 10 min at 72°C.

Plasmids containing promoters for expression of F1-antigen were then produced. The promoters for the *phoP, pagC* and *ompC* genes have previously been mapped and upstream regulatory regions identified (Soncini F C, et al., J. Bacteriol. 1995;177:4364-71, Pulkkinen W S, et al., J. Bacteriol. 1991;173:86-9, Puente J L, et al., Gene. 1987;61:75-83, Puente J L, et al., Gene. 1989;83:197-206). For the *phoP* gene promoter a 139bp DNA fragment was identified which included the *phoPp1* and *phoPp2* gene promoters and 80 bp upstream of the -35 site which has been predicted to form step loop structures (Soncini F C, et al., J. Bacteriol. 1995;177:4364-71). For the *pagC* gene promote a 715 bp DNA fragment included 125 bp upstream of the -35 region (Pulkkinen W S, et al., J. Bacteriol. 1991;173:86-9). For the *ompC* gene promoter a 371 bp DNA fragment included a 275 bp region upstream of the -35 region (Puente J L, et al., Gene. 1987;61:75-83, Puente J L, et al., Gene. 1989;83:197-206).

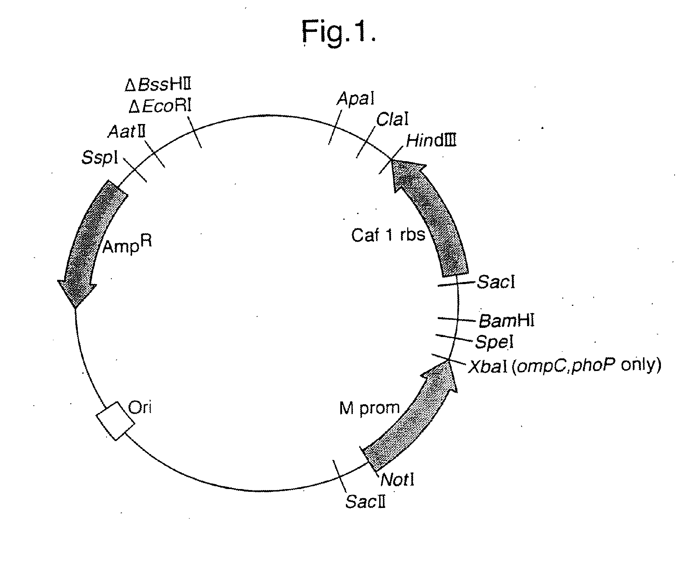

These DNA fragments were amplified from S. *typhimurium* strain SL1344 genomic DNA using the PCR. For comparison with a constitutive gene promoter, a 196 bp DNA fragment encoding the *lacZ* gene promoter and 140 bp upstream of the -35 region was identified. The 3' end of all of the DNA fragments terminated before the SD regions associated with the genes. These promoters were cloned upstream of the *caf1* open reading frame (encoding the *Y. pestis* F1-antigen) and SD region in a low copy number vector (pBR322; Fig 1) and the recombinant plasmids (pP*_{ompC}*-F1, pP*_{phcP}*-F1, pP*_{pagC}*-F1 or pP*_{lacZ}-*F1) transformed into *S*. *typhimurium* SL3261 (*aro*A).

Oligonucleotide primers were designed to amplify promoter regions using the PCR (Table 1).

**Table 1**

| Oligonucleotide pair | SEQ ID No | Prom |
|---|---|---|
| 5' AAGGAAAAAAGCGGCCGCCGCCCAATACGCAAACCG 3' | 5 | |
| 5' GAATTCACTAGTATTGTTATCCGCGCTCACAAT 3' | 6 | P*lac* |
| | | |
| 5' AAGGAAAAAAGCGGCCGCTGACTCTGGTCGACGAACTTA 3' | 7 | |
| 5' CTAGTCTAGATGTGTTAACCAATAAGAACAGTCTA 3' | 8 | *PphoP* |
| | | |
| 5' AAGGAAAAAAGCGGCCGCTAAACAGACATTCAGAAGTGAATG3' | 9 | |
| 5' CTAGTCTAGAATATGCCTTTATTGCTTTTTTATG 3' | 10 | *PompC* |
| | | |
| 5' AAGGAAAAAAGCGGCCGCGTTAACCACTCTTAATAATAATG 3' | 11 | |
| 5' CTAGACTAGTTACTACTTATTATTTACG 3' | 12 | *PpagC* |

| | | |
|---|---|---|
| Restriction sites are shown underlined. | | |

The primers included unique *Not*1*,* Xbal or Spel sites. Regions amplified included the -10 and -35 regions and upstream regulatory binding sites, but excluded the Shine-Dalgarno (SD) ribosome binding site.

After PCR amplification of the promoter regions from *S*. *typhimurium* SL 1344 template DNA (or plasmid pUC18 template DNA for amplification of the lac promoter), the DNA fragments were purified using Microcon 100 centrifugal concentrations (Millipore, Watford, UK). The purified DNA fragments were cloned into suitable digested plasmid pBluescript SK-, electroporated into *E*. *coli* JM101 and the cloned fragments were nucleotide sequenced to ensure their authenticity. After digestion of the recombinant plasmids with Sacl and BssHll and agarose gel electrophoresis, DNA fragments containing the promoter regions were purified using Qiaex (Qiagen Ltd, Crawley, UK) and blunt ended using Klenow fragment (Sambrook J, Frtisch E F, Maniatis T. 1989. Molecular Cloning: A Laboratory Manual, 2^{nd} ed. Cold Spring Harbor Laboratory press, New York).

The authentic promoter sequences were then cloned into plasmid pBR322 which had been digested with *Eco*Rl and *Nru*l and then blunt ended using Klenow fragment (Sambrook J, Frtisch E F, Maniatis T. 1989. Molecular Cloning: A Laboratory Manual, 2^{nd} ed. Cold Spring Harbor Laboratory press, New York). The orientation of the cloned DNA fragment in the plasmid was determined by analysing, using agarose gel electrophoresis, the DNA fragments obtained after digestion with *Xba*l, *Ssp*l or *Sty*l.

A DNA fragment which encoded the Caf1 open reading frame and the ribosome binding site was isolated after digestion of plasmid pORF1 (Oyston P C F, et al., Infect. Immun. 1995;63:563-68) with *Eco*Rl followed by blunt ending of the DNA and further digestion with *Hind*lll*.* The purified DNA fragment was ligated with promoter plasmids with which had been digested with *Sma*l and *Hind*lll. The final recombinant plasmids were transformed into *E. coli* strain JM109.

Plasmids were isolated from *E*. *coli* (Sambrook J, Frtisch E F, Maniatis T. 1989. Molecular Cloning: A Laboratory Manual, 2^{nd} ed. Cold Spring Harbor Laboratory press, New York) and electroporated into S. *typhimurium* SL3281 (aroA) after passage through *S*. *typhimurium* LB5010 to ensure methylation of the DNA.

### Example 2

The stability of the different plasmids encoding F1-antigen driven from different promoters in *S*. *typhimurium* SL3261 was determined after culture of the bacteria in L-broth for 24hr (*in vitro* stability) and enumeration of bacteria which grew on L-agar or L-agar containing amplicillin (Sambrook J, Frtisch E F, Maniatis T. 1989. Molecular Cloning: A Laboratory Manual, 2^{nd} ed. Cold Spring Harbor Laboratory press, New York). The results indicated that all of the plasmids were retained by at least 80% of the bacteria which had been cultured *in vitro* (pP*_{ompC}*-F1, 83%; pP*_{phoP}*-F1, 100%; pP*_{pagC}*-F1, 98%; pP*_{lacZ}*-F1, 95%) .

### Example 3

### Colonisation of host tissues

The *in vivo* stability of plasmids was determined by inoculating groups of 10 female BALB/c mice orally with 10⁹ cfu of bacteria in 100µl of PBS, and enumerating bacteria isolated 11 days later from homogenised spleen tissue on L-agar or L-agar + ampicillin.

In the case of the P_{*pag*C}*-*F1 construct and the *S*. *typhimurium* SL3261 control strain, bacteria were also cultured from mesenteric lymph nodes (10/mouse) each homogenised in 2ml of PBS or from homogenised liver tissue which were removed on. 11 days after dosing. Bacteria were enumerated bacteria as described above.

With the exception of the bacteria containing the P*_{pagC}*-F1 plasmid. and the SL3261 control bacteria, ampicillin-resistant bacteria could be recovered from all of the spleens isolated from orally dosed mice. The *in vivo* stability of all of the plasmids within *Salmonella* was lower than the stability of the plasmids within *Salmonella* cultured *in vitro.*

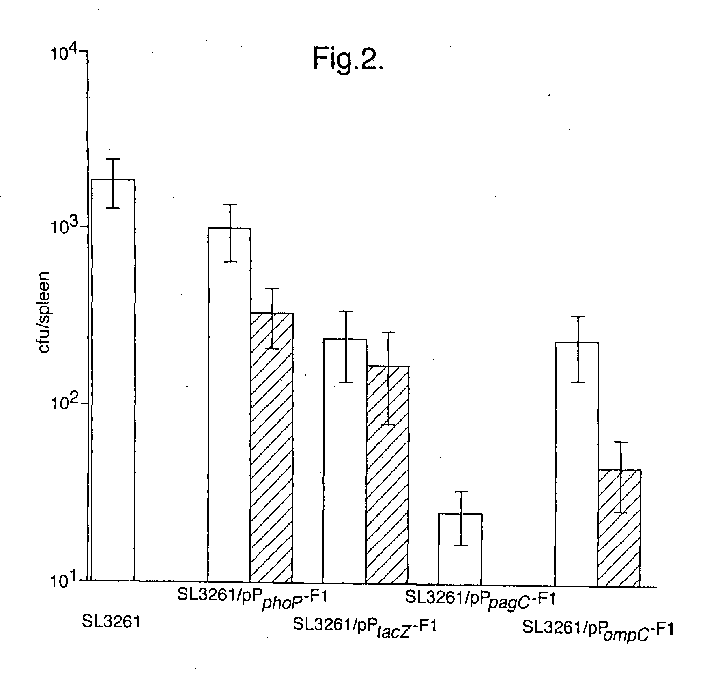

Although mice were dosed orally with similar numbers of bacteria there were marked differences in the level of colonisation of spleen tissues at day 11 (Fig 2). The highest levels of colonisation were found with bacteria containing pP*_{phoP}*-F1. Low numbers of *Salmonella* containing pP*_{pagC}*-F1 were recovered from spleen tissues (maximum 50 cfu/spleen) and none of the bacterial colonies were ampicillin resistant. To investigate why the presence of the pP*_{pagC}*-F1 affected the colonising ability of this *Salmonella,* a more detailed study was undertaken which involved the enumeration of bacteria in the liver and in mesenteric lymph nodes 11 days after oral dosing. The levels of SL3261 containing pP_{*pag*C}-F1 isolated from liver tissues were similar to those isolated from spleens (data not shown). Whereas the mean number of SL3261 isolated from mesenteric lymph nodes was 2.2x10³ cfu, SL3261 containing pP*_{pagC}*-F1 could be isolated only at low level (mean 20 cfu) from these tissues.

### Example 4

### Serum antibody responses to F1-antigen

Groups of 5 or 8 female BALB/c mice were immunised via intragastric intubation on days 0 and 14 with 1 x 10⁹ cells of the P*_{ompC}-*F1*,* P*_{phoP}*-F1, P*_{pagC}*-F1 or P*_{lacZ}-*F1 constructs, or the control *S*. *typhimurium* strain, SL3261, in 0.1 ml of phosphate-buffered saline (PBS). Bacteria were grown statically overnight at 37°C. All oral inoculations were carried out with a stainless steel gavage needle without an anaesthetic. The inoculum dose was verified by plating serial dilutions of each culture on L-agar plates with or without ampicillin.

On days 21, 28 and 98 mice were anaesthetized by intraperitoneal (i.p.) administration of a cocktail of domitor (6 mg per dose) and Ketalar (27mg per dose) and blood was collected by cardiac puncture. Mice were then sacrificed by cervical dislocation. Blood was allowed to clot at 4°C overnight prior to centrifugation (10,000 x g, 10 min, 4°C) and the serum stored at -20°C until tested.

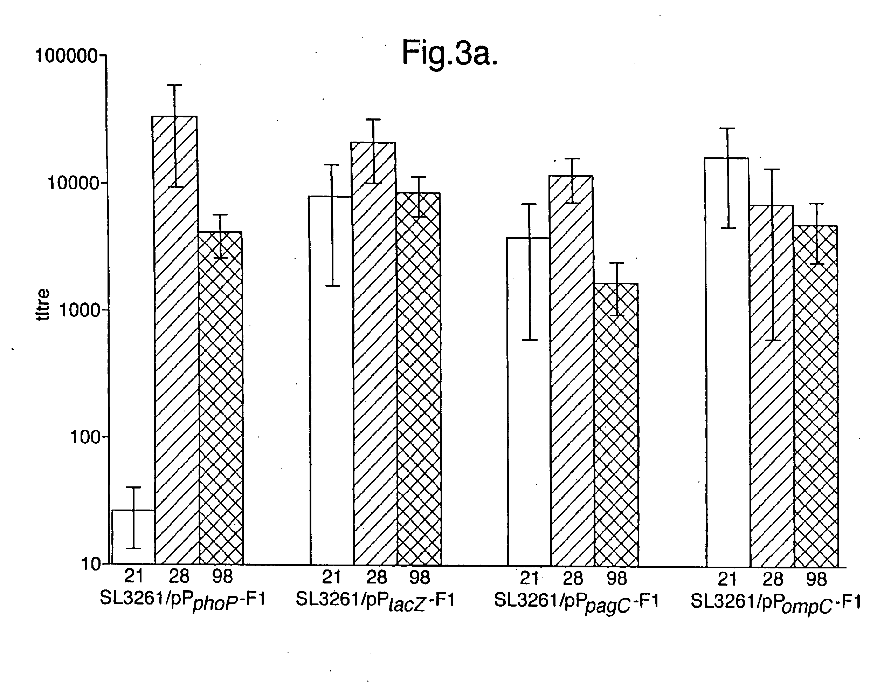

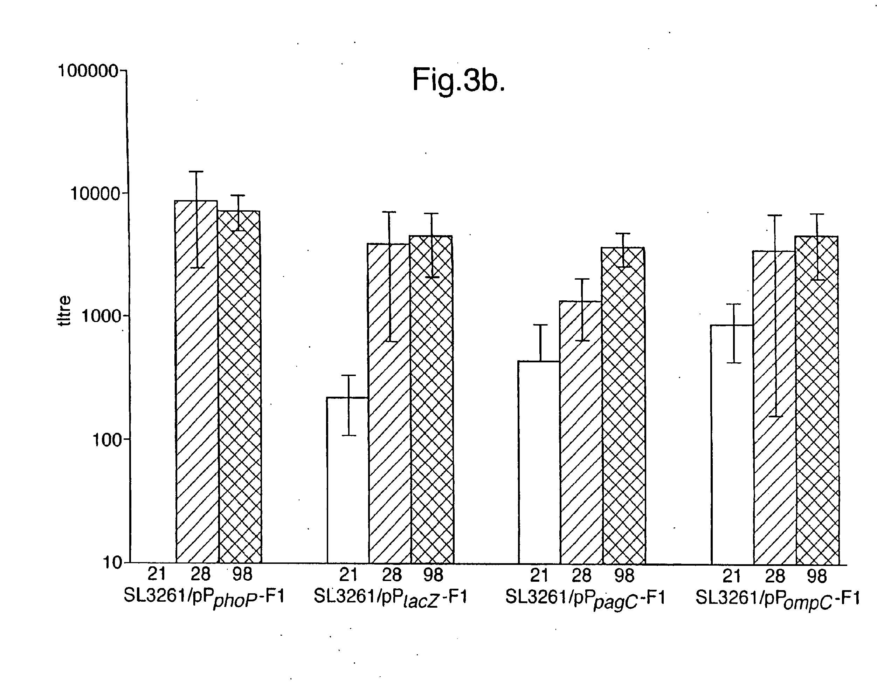

After i.g. dosing with the recombinant *Salmonella*, mice in all groups developed IgG serum antibody to the carrier bacterium, which reached a maximum level 98 days after immunisation (Fig 3a). In the case of SL3261 expressing P*_{phoP}*-F1,the onset of the immune response was delayed. All groups of mice developed IgG serum antibody to F1-antigen (Fig 3b), which had reached a peak at day 28 and declined by day 98. There was no significant difference in the peak titres to F1-antigen induced by the different recombinant *Salmonella.*

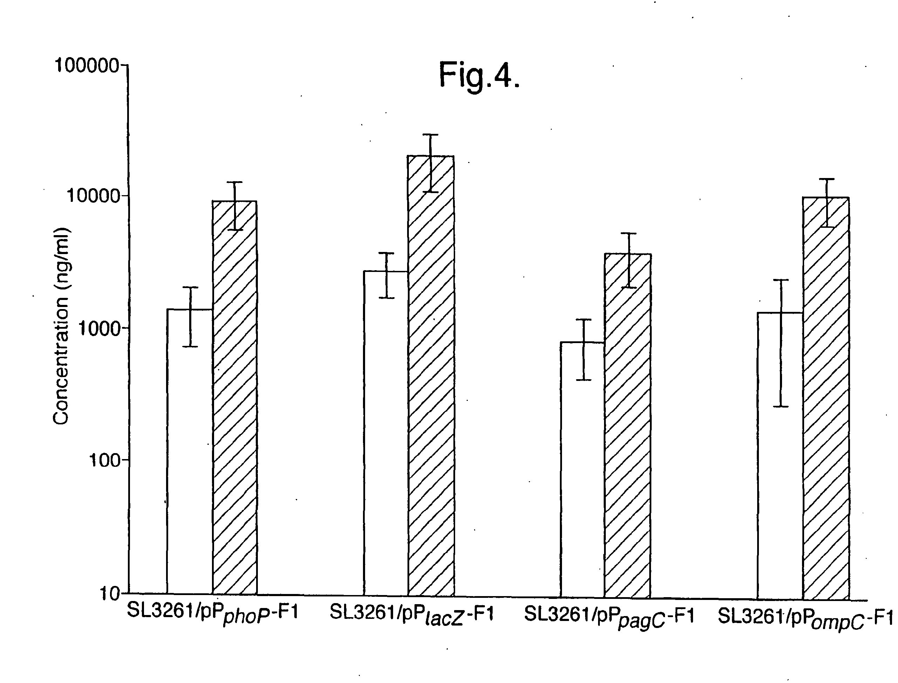

When the isotype of this antibody was determined on day 98, it was found to be predominantly of an IgG2ₐ isotype in all groups of immunised animals (Fig 4), suggesting that a Th1-type response was induced. Several other workers have shown that recombinant S. typhimurium induce a Th1-type response to the guest antigen (Brett S J, et al., Immunology 1993;80:306-12, Thatte J, et al., Int. Immunol. 1993;5:1431-36).

### Example 5

### Mucosal antibody responses to F1-antigen

The ability of the different recombinant *Salmonella* to induce a mucosal antibody response after i.g. dosing was determined by measuring the levels of circulating IgA antibody to F1-antigen or the levels of IgA antibody to F1-antigen in gut or lung wash samples. After dosing as described in Example 4, on days 21 and 28, gut and lung wash samples were collected. Briefly, gut wash samples were collected by resecting a 10 cm length of small intestine and flushing with 5 ml of PBS. Samples were sonicated for 0.5 min prior to centrifugation (12,000 x g, 30 mins 4°C) and the supernatant was decanted and lyophilized. Broncho-alveolar washings were collected from individual animals by injecting 5 ml of chilled lavage medium (0.9% (w/v) NaCl, 0.05% (v/v) tween 20, 0.1% (w/v) NaN₃ and 1mM phenylmethylsulfonyl fluoride) into the trachea using an intravenous canula and inflating the lungs. A syringe was used to remove the washings, which were subsequently centrifuged (12,000 x g, 30 min, 4°C) prior to lyophilisation of the supernatant fluid. Gut and lung wash samples were reconstituted in 200µl sterile water immediately before use.

All measurements of antibody levels in individual animals were determined in duplicate. For enzyme-linked immunosorbant assays (ELISAs) to determine IgG and IgA titres, 96-well microtiter plates were coated overnight at 4°C either with 50µl 5µg/ml purified F1-antigen (Miller J, et al., FEMS Microbiology and Immunology 1998;21:213-21) in PBS or with 50µl 6gg/ml *S*. *typhimurium* SL3261 lysate in PBS, prepared as follows. Bacteria were grown statically overnight at 37°C, prior to harvesting and resuspension in PBS to an approximate concentration of 1 x 10¹⁰ cfu/ml. Cells were heat-killed in a boiling water bath for 30 min, cooled on ice and then sonicated on ice for 6 pulses of 30 s. Total protein concentration was determined by a BCA protein assay (Pierce and Warriner, Chester, UK). Plates were blocked for 1 h at 37°C with PBS containing 1% (w/v) skimmed milk powder (BLOTTO). Serum, gut and lung wash samples were diluted in _ BLOTTO and 50µl volumes were assayed in duplicate in a series of twofold dilutions. After incubation overnight at 4°C, plates were washed three times in PBS with 0.02% (v/v) tween 20. Peroxidase-conjugated secondary antibodies against mouse IgG or IgA (Harlan Sera-Lab Ltd, Loughborough, UK), diluted 1:2000 in BLOTTO were incubated for 1 h at 37°C. The plate was washed as previously and 100µl of 2,2'-azino bis(3-ethylbenzthiazoline-6-sulfonic acid) substrate (ABTS; Sigma, Poole, UK) was added. Antibody titre was estimated as the maximum dilution of serum giving an absorbance₄₁₄ₙₘ reading 0.1 U above background (Sera from animals immunised with SL3261 alone).

To determine IgG₁, or IgG₂ₐ concentrations, ELISAs were performed essentially as above, except that wells were coated with 10µg/ml anti-mouse IgG (Fab-specific, Sigma, Poole, UK) 5µg/ml purified F1-antigen in PBS or 6µg/ml S. typhimurium SL3261 lysate. Purified IgG₁ or IgG₂ₐ (Sigma, Poole, UK) and day 98 serum samples were diluted in BLOTTO. Peroxidase-labelled secondary antibodies against mouse IgG₁ or IgG₂ₐ were diluted 1:4000 BLOTTO before use.

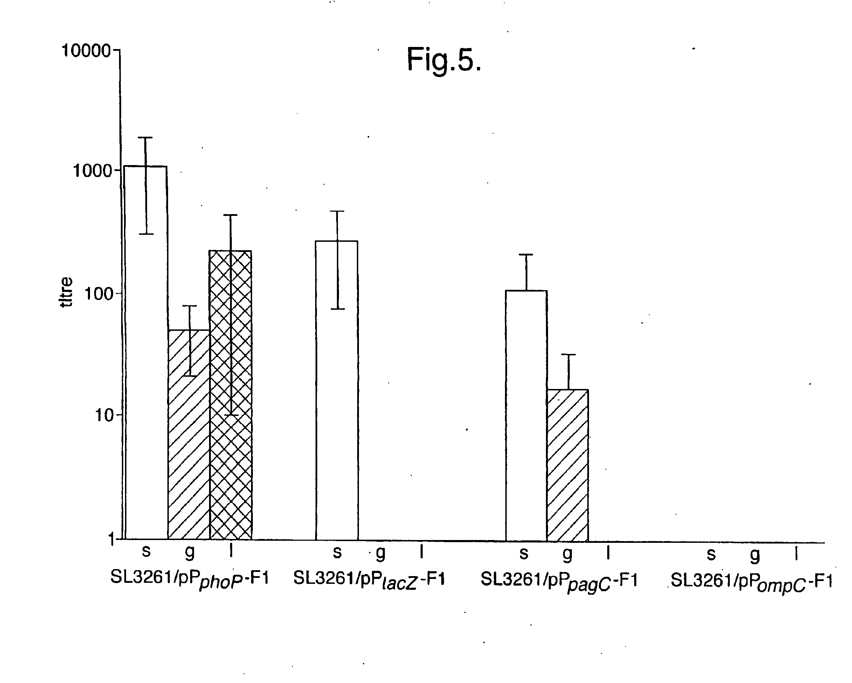

The results (Fig 5) indicated that SL3261 containing pP*_{phoP}*-F1, pP*_{lacZ}*-F1 or pP*_{pagC}*-F1 plasmids all induced serum IgA antibody to F-antigen. The induction of circulating IgA to F1-antigen did not correlate with the presence of IgA to F1-antigen at mucosal surfaces. For example, SL3261 / pP*_{lacZ}*-F1 induced high levels of serum antibody to F1-antigen but IgA antibody to F1-antigen was not detected in gut or lung wash samples. Only SL3261 / pP*_{phoP}*-F1 induced an IgA antibody response to F1-antigen in both the gut and the lung.

### Example 6

### Production of IgA by Peyer's patch cells

Peyer's patches were also removed to determine the presence of Fl- and Salmonella specific IgA producing cells in the gut. Briefly, a total of 8 Peyer's patches were removed from 5 mice in each treatment group (see Example 4) and pooled. Cells were separated by crushing through a cell strainer, washed by centrifugation and resuspended in 1.4 ml of Dulbeccos Modified Eagles Medium (DMEM) + 10% foetal calf serum (FCS). Duplicate samples (100µl/well) were then plated onto plates previously coated with 5µl/ml F1 or 6µg/ml S. typhimurium SL3261 lysate and blocked with 20% FCS in DMEM, and incubated for 48hours at 37°C. Plates were washed and incubated for 1 hour with peroxidase-labelled secondary antibody against mouse IgA, diluted 1:2000 in PBS before use, and developed with 100µl of ABTS.

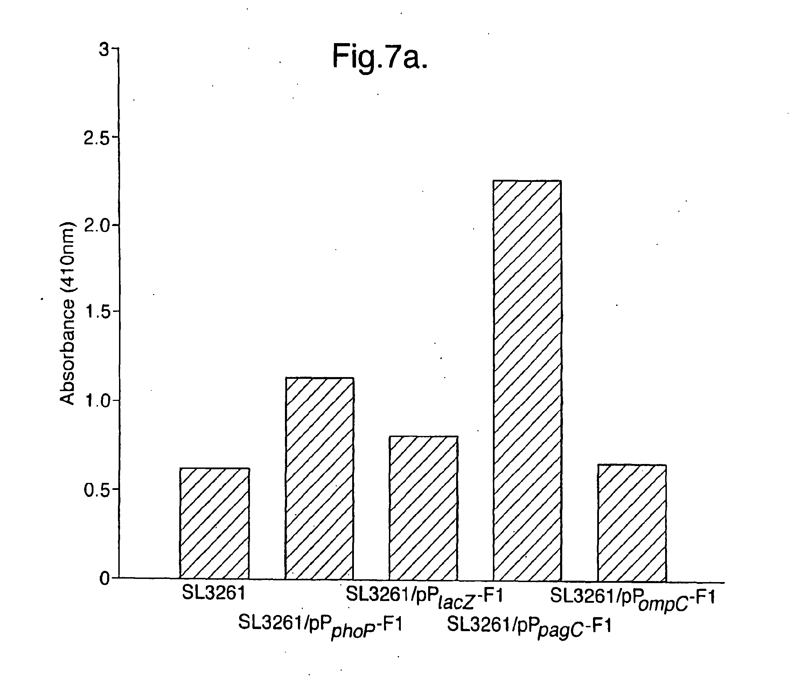

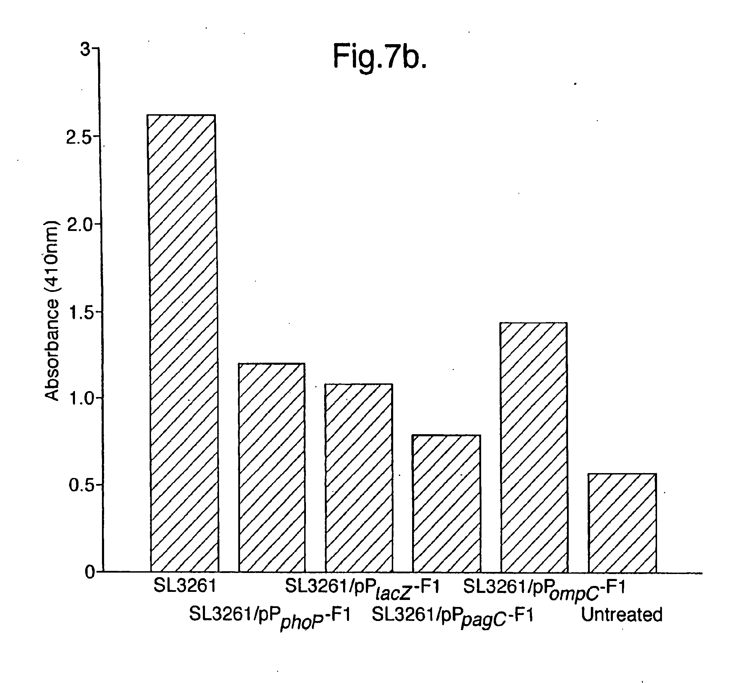

The results are shown in Figure 7. These show showed that the secretion of IgA against F1-antigen was greatest in Peyer's patch cells isolated from mice which had been immunised with SL3261 containing pP*_{pagC}*-F1 (Fig 7a). Peyer's patch cells taken from mice which had been immunised with SL3261 / pP*_{phoP}*-F1 also produced IgA. Cells taken from other groups produced only low levels of IgA to F1-antigen.

This pattern of response was not reflected in the pattern of production of IgA against *Salmonella;* Peyer's patch cells taken from mice which had been immunised with SL3261 / pP_{*pag*C}-F1 produced only low levels of antibody to *Salmonella* (Fig 7b).

## Claims

1. A method of enhancing expression of a desired protein at mucosal effector sites, said method comprising placing the protein to be expressed under the control of a promoter having SEQ ID NO 2 or SEQ ID NO 3 or a fragment or variant having at least 60% homology or any of these which has promoter activity, and causing expression in mucosal cells.

2. A construct comprising a promoter selected from the P_{phoP} and P_{pagC} or fragments or variants thereof which can act as promoters, operatively interconnected with a nucleic acid which encodes a protein, able to induce a protective immune response against an organism, in a mammal to which it is administered, wherein said construct contains no further elements of the phoP or pagC gene.

3. A recombinant gut-colonising microorganism which has been transformed with a construct according to claim 2.

4. A recombinant gut-colonising microorganism according to claim 3 wherein said protein is heterologous to said microorganism.

5. A recombinant gut-colonising microorganism according to claim 3 or claim 4 wherein the promoter is P_{phoP} promoter.

6. A recombinant gut-colonising microorganism according to claim 3 or claim 4 wherein the promoter is P_{pagC} promoter.

7. A recombinant gut-colonising microorganism according to any one of claims 3 to 6 which comprises a *Salmonella spp.*

8. A recombinant gut-colonising microorganism according to claim 7 wherein the *Salmonella spp.* is *Salmonella typhimurium* or *Salmonella typhi.*

9. A recombinant gut-colonising microorganism according to any one claims 3 to 8 wherein the gut-colonising microorganism is attenuated.

10. A construct according to claim 2 or a recombinant gut-colonising microorganism according to any one of claims 3 to 9 wherein the heterologous protein, is able to induce a protective immune response against *Yersinia pestis.*

11. A construct or a recombinant gut-colonising microrganism according to claim 10 wherein the said heterologous protein comprises an F1-antigen of *Yersinia pestis* or an antigenic fragment or variant thereof.

12. A vaccine comprising a recombinant gut-colonising microorganism according to any one claims 3 to 11.

13. A vaccine according to claim 12 which further comprises a pharmaceutically acceptable carrier or diluent.

14. A vaccine according to claim 12 or claim 13 which is adapted for oral administration.

15. A construct according to claim 2 for use in the production of a protective immune response in mucosal effector cells.

16. The use of a promoter selected from P_{phoP} and P_{pagC} in the production of a vaccine comprising a recombinant gut-colonising organism.

## Patentansprüche

1. Verfahren zur Verstärkung der Expression eines gewünschten Proteins an Schleimhaut-Effektorstellen, wobei das Verfahren umfaßt, daß
das zu exprimierende Protein unter die Kontrolle eines Promotors der Sequenz SEQ ID NO 2 oder SEQ ID NO 3 oder eines Fragments oder einer Variante mit mindestens 60 % Homologie oder irgendeines Stoffs davon mit Promotor-Aktivität gebracht wird
und die Expression in Schleimhautzellen bewirkt wird.

2. Konstrukt, das einen Promotor enthält, der ausgewählt ist unter P_{phoP} und P_{pagC} oder Fragmenten oder Varianten davon, die als Promotoren wirken können, und operativ mit einer Nucleinsäure verbunden ist, die ein Protein codiert, das befähigt ist, eine schützende Immunantwort gegen einen Organismus in einem Säuger zu induzieren, dem es verabreicht wird, wobei das Konstrukt keine weiteren Elemente des Gens phoP oder des Gens pagC enthält.

3. Rekombinanter, den Darm besiedelnder Mikroorganismus, der mit einem Konstrukt nach Anspruch 2 transformiert wurde.

4. Rekombinanter, den Darm besiedelnder Mikroorganismus nach Anspruch 3, wobei das Protein bezüglich des Mikroorganismus heterolog ist.

5. Rekombinanter, den Darm besiedelnder Mikroorganismus nach Anspruch 3 oder 4, wobei der Promotor der Promotor P_{phoP} ist.

6. Rekombinanter, den Darm besiedelnder Mikroorganismus nach Anspruch 3 oder 4, wobei der Promotor der P_{pagC} ist.

7. Rekombinanter, den Darm besiedelnder Mikroorganismus nach einem der Ansprüche 3 bis 6, der eine *Salmonella spp.* ist.

8. Rekombinanter, den Darm besiedelnder Mikroorganismus nach Anspruch 7, wobei die *Salmonella spp. Salmonella typhimurium* oder *Salmonella typhi* ist.

9. Rekombinanter, den Darm besiedelnder Mikroorganismus nach einem der Ansprüche 3 bis 8, wobei der den Darm besiedelnde Mikroorganismus abgeschwächt ist.

10. Konstrukt nach Anspruch 2 oder rekombinanter, den Darm besiedelnder Mikroorganismus nach einem der Ansprüche 3 bis 9, wobei das heterologe Protein befähigt ist, eine schützende Immunantwort gegen *Yersinia pestis* zu induzieren.

11. Konstrukt oder rekombinanter, den Darm besiedelnder Mikroorganismus nach Anspruch 10, wobei das heterologe Protein ein F1-Antigen von *Yersinia pestis* oder ein antigenes Fragment oder eine antigene Variante davon umfasst.

12. Impfstoff, der einen rekombinanten, den Darm besiedelnden Mikroorganismus nach einem der Ansprüche 3 - 11 enthält.

13. Impfstoff nach Anspruch 12, der ferner einen pharmazeutisch akzeptablen Träger oder ein pharmazeutisch akzeptables Verdünnungsmittel enthält.

14. Impfstoff nach Anspruch 12 oder 13, der zur oralen Verabreichung hergerichtet ist.

15. Konstrukt nach Anspruch 2 zur Verwendung bei der Erzeugung einer schützenden Immunantwort in Schleimhaut-Effektorzellen.

16. Verwendung eines unter P_{phoP} und P_{pagC} ausgewählten Promotors bei der Herstellung eines Impfstoffs, der einen rekombinanten, den Darm besiedelnden Organismus enthält.

## Revendications

1. Procédé d'amélioration de l'expression d'une protéine souhaitée au niveau de sites effecteurs muqueux, ledit procédé comprenant le fait de placer la protéine à exprimer sous le contrôle d'un promoteur ayant la SEQ ID n°2 ou la SEQ ID n°3 ou un fragment ou une variante ayant au moins 60 % d'homologie ou toute autre qui a une activité de promoteur, et le fait de provoquer l'expression dans des cellules muqueuses.

2. Produit comprenant un promoteur choisi parmi PphoP et PpagC ou des fragments ou variantes de ceux-ci qui peuvent agir comme promoteurs, interconnectés de façon opérationnelle avec un acide nucléique qui code pour une protéine, capable d'induire une réponse immunitaire protectrice contre un organisme, chez un mammifère auquel il est administré, dans lequel ledit produit ne contient pas d'autres éléments du gène phoP ou pagC.

3. Microorganisme recombinant colonisant l'intestin qui a été transformé avec un produit selon la revendication 2.

4. Microorganisme recombinant colonisant l'intestin selon la revendication 3, dans lequel ladite protéine est hétérologue audit organisme.

5. Microorganisme recombinant colonisant l'intestin selon la revendication 3 ou la revendication 4, dans lequel le promoteur est un promoteur P_{phoP}.

6. Microorganisme recombinant colonisant l'intestin selon la revendication 3 ou la revendication 4, dans lequel le promoteur est un promoteur P_{pagC}.

7. Microorganisme recombinant colonisant l'intestin selon l'une quelconque des revendications 3 à 6, qui comprend une espèce de salmonelle.

8. Microorganisme recombinant colonisant l'intestin selon la revendication 7, dans lequel l'espèce de salmonelle est Salmonella Typhimurium ou Salmonella typhi.

9. Microorganisme recombinant colonisant l'intestin selon l'une des revendications 3 à 8 dans lequel le microorganisme colonisant l'intestin est atténué.

10. Produit selon la revendication 2 ou microorganisme recombinant colonisant l'intestin selon l'une des revendications 3 à 9 dans lequel la protéine hétérologue est capable d'induire une réponse immunitaire protectrice contre Yersinia pestis.

11. Produit ou microorganisme recombinant colonisant l'intestin selon la revendication 10, dans lequel ladite protéine hétérologue comprend un antigène F1 de Yersinia pestis ou un fragment ou une variante antigénique de celui-ci.

12. Vaccin comprenant un microorganisme recombinant colonisant l'intestin selon l'une des revendications 3 à 11.

13. Vaccin selon la revendication 12 qui comprend en outre un véhicule ou diluant pharmaceutiquement acceptable.

14. Vaccin selon la revendication 12 ou la revendication 13 qui est adapté pour une administration orale.

15. Produit selon la revendication 2 pour une utilisation dans la production d'une réponse immunitaire protectrice dans des cellules effectrices muqueuses.

16. Utilisation d'un promoteur choisi parmi PphoP et PpagC dans la production d'un vaccin comprenant un organisme recombinant colonisant l'intestin.


### SEQUENCE LISTING

<110> The Secretary of State for Defence, Defence Evaluation and Research Agency
   Titball, Richard W
   Bullifent, Helen L
<120> Recombinant Microorganisms
<130> P1246/WOD
<140> PCT/GB00/03402
   <141> 2000-09-06
<150> GB 0017000.1
   <151> 2000-07-12
<150> GB 9921275.5
   <151> 1999-09-10
<160> 12
<170> PatentIn version 3.0
<210> 1
   <211> 196
   <212> DNA
   <213> Salmonella typhimurium
<400> 1
   cgcccaatac gcaaaccgcc tctccccgcg cgttggccga ttcattaatg cagctggcac 60
   gacaggtttc ccgactggaa agcgggcagt gagcgcaacg caattaatgt gagttagctc 120
   actcattagg caccccaggc tttacacttt atgcttccgg ctcgtatgtt gtgtggaatt 180
   gtgagcggat aacaat 196
<210> 2
   <211> 139
   <212> DNA
   <213> Salmonella typhimurium
<210> 3
   <211> 715
   <212> DNA
   <213> Salmonella typhimurium
<400> 3
   gttaaccact cttaataata atgggtttta tagcgaaata gactttttta tcgcgtgttc     60
   aatatttgcg ttagttatta tttttttgga atgtaaattc tctctaaaca caggtgatat    120
   ttatgttgga attgtggtgt tgattctatt cttataatat aacaagaaat gttgtaactg   180
   atagatatat taaaagatta aatcggagcg ggaataaagc gtgctaagca tcatcgtgaa   240
   tatgattaca gcgcctgcga tggcatataa ccgtattgcg gatggagcgt cacgtgagga   300
   ctgtgaagca caatgcgata tgttctgatt atatggcgag tttgcttaat gacatgtttt   360
   tagccgaacg gtgtcaagtt tcttaatgtg gttgtgagat tttctcttta aatatcaaaa   420
   tgttgcatgg gtgatttgtt gttctatagt ggctaaacac tttatggttt ctgttaaata   480
   tatatgcgtg agaaaaatta gcattcaaat ctataaaagt tagatgacat tgtagaaccg   540
   gttacctaaa tgagcgatag agtgcttcgg tagtaaaaat atctttcagg aagtaaacac   600
   atcaggagcg atagcggtga attattcgtg gttttgtcga ttcggcatag tggcgataac   660
   tgaatgccgg atcggtactg caggtgttta aacacaccgt aaataataag tagta        715
<210> 4
   <211> 371
   <212> DNA
   <213> Salmonella typhimurium
<400> 4
   taaacagaca ttcagaagtg aatgacggta ataaataaag ttaatgatga tagcggtcac 60
   tattttagtt gcgaatgaag attctgtttt atcattcagt gctatgaatt tcatcaattt 120
   aacccgttga ttttaaaagt ttcgtgaaat atattttgtc tatttgtgct tatttttact 180
   tgatttttgc tttaaaaaag ttccgtaaaa ttcatatttt gaaacatcta tatagataac 240
   tgtaacatct taaaagtttt agtatcatat tcgtgttgga ttattctgta tttttgcgga 300
   gaatggactt gccgactggt taatgagggt taaccagtaa gcagtggcat aaaaaagcaa 360
   taaaggcata t 371
<210> 5
   <211> 36
   <212> DNA
   <213> Artificial
<220>
   <223> Oligonucleotide
<400> 5
   aaggaaaaaa gcggccgccg cccaatacgc aaaccg 36
<210> 6
   <211> 33
   <212> DNA
   <213> Artificial
<220>
   <223> Oligonucleotide
<400> 6
   gaattcacta gtattgttat ccgcgctcac aat 33
<210> 7
   <211> 39
   <212> DNA
   <213> Artificial
<220>
   <223> Oligonucleotide
<400> 7
   aaggaaaaaa gcggccgctg actctggtcg acgaactta 39
<210> 8
   <211> 35
   <212> DNA
   <213> Artificial
<220>
   <223> Oligonucleotide
<400> 8
   ctagtctaga tgtgttaacc aataagaaca gtcta 35
<210> 9
   <211> 42
   <212> DNA
   <213> Artificial
<220>
   <223> Oligonucleotide
<400> 9
   aaggaaaaaa gcggccgcta aacagacatt cagaagtgaa tg 42
<210> 10
   <211> 34
   <212> DNA
   <213> Artificial
<220>
   <223> Oligonucleotide
<400> 10
   ctagtctaga atatgccttt attgcttttt tatg 34
<210> 11
   <211> 41
   <212> DNA
   <213> Artificial
<220>
   <223> Oligonucleotide
<400> 11
   aaggaaaaaa gcggccgcgt taaccactct taataataat g 41
<210> 12
   <211> 28
   <212> DNA
   <213> Artificial
<220>
   <223> Oligonucleotide
<400> 12
   ctagactagt tactacttat tatttacg 28